# EUROPEAN PATENT APPLICATION

(11) **EP 4 483 842 A1**
(43) Date of publication of application: **01.01.2025**
(21) Application number: 22927173.9
(22) Date of filing: 21.02.2022
(51) Int. Cl.: A61F 2/02, A61B 17/00

(54) **HEART NET PLACEMENT TOOL AND PLACEMENT METHOD**

(71) Applicant: iCorNet Laboratory Co., Ltd., Nagoya-shi, Aichi, 464-0858 (JP)
(72) Inventor: AKITA Toshiaki, Nagoya-shi, Aichi 464-0858 (JP); SASAKI Toshiya, Nagoya-shi, Aichi 464-0858 (JP)
(74) Representative: Wenzel Nemetzade Warthmüller Patentanwälte Part mbB
(86) International application number: PCT/JP2022/006827
(87) International publication number: WO 2023/157272

(57) **Abstract**

One object is to enable a cardiac net to be mounted with reducing invasion. A mounting device 100 configured to support a cardiac net 10 is provided. The cardiac net 10 has a tubular portion that is formed over the entire outer circumference of an opening provided on an upper portion of the cardiac net 10 by folding back the opening. The mounting device 100 has a first part and a second part and is configured to support the cardiac net 10 by inserting curved portions at front edges of the respective parts into the tubular portion of the cardiac net 10. The respective ends of the mounting device on a side which the cardiac net 10 is attached to are opened and closed like scissors by opening and closing handles of the mounting device. This configuration accordingly enables the cardiac net 10 to be inserted in the closed state into the body of a patient. The first part and the second part are configured to be separable from each other. This separable structure enables the respective parts to be separated from each other and taken out individually after mounting of the cardiac net 10. This configuration enables the cardiac net 10 to be mounted with reducing the invasion to the patient.

## Description

### Technical Field

The present disclosure relates to a mounting device of a cardiac net and a method of mounting a cardiac net.

### Background

Mounting of a cardiac net to the heart is performed as a treatment of heart diseases. The cardiac net mainly used for cardiac dilatation is provided with no electrodes. The cardiac net mainly used for the purpose of, for example, defibrillation, ventricular pacing, and sensing of the heart motions is provided with electrodes.

The following techniques have been disclosed with regard to a mounting device used to mount the cardiac net to the heart.

Patent Literature 1 discloses a mounting device configured to support a cardiac net by a plurality of openable and closable support portions. This mounting device is inserted in a closed state into the body of a patient from below the heart of the patient and is subsequently opened to mount the cardiac net to the heart. This mounting device is then closed again and is pulled out of the body. This mounts the cardiac net to the heart.

Patent Literature 2 discloses a mounting device configured to support a cardiac net by a pair of support portions having curved front edges. This mounting device enables the cardiac net to be inserted into the body from a median or a side of the breast of the patient and then enables the cardiac net to be mounted to the heart in such a manner as to scoop the heart.

### Citation List

### Patent Literature

Patent Literature 1: Japanese Patent No. 2014-166234A
Patent Literature 2: Design Registration No. 1666593B

### Summary

### Technical Problem

Mounting of the cardiac net to the heart causes invasion to the patient. It is preferable to minimize the degree of invasion. The mounting device described in Patent Literature 1 is, however, inserted from below the heart and requires thoracotomy of the patient. This tends to cause a heavy invasion. It is also not desirable that a surgical scar is left in the vicinity of the center of the body.

The mounting device described in Patent Literature 2, on the other hand, causes the cardiac net to be mounted to the heart in such a manner as to scoop the heart by the mounting device, in the case where the cardiac net is inserted not from the median of the breast but is inserted from the side of the patient. This avoids the problem that the surgical scar is left in the vicinity of the center of the body. This, however, needs an operation from the limited operative wound and thereby requires a mechanism of allowing for detachment of the mounting device with following the heart shape not to damage the heart in the process of detachment, while requiring the sufficient rigidity in the process of attachment. An integral type has a fixed shape and accordingly has a difficulty in balancing between the rigidity in the process of attachment and the flexibility in the process of detachment. More specifically, a front edge of the mounting device is curved inward. The integral type accordingly needs an operation like scratching the heart for detachment and thereby requires to maintain the flexibility. There is accordingly a limitation in increasing the rigidity in the process of attachment.

By taking into account the problems described above, an object of the present disclosure is to provide a technique that satisfies two conflicting requirements in the process of mounting the cardiac net to the heart, i.e., both the rigidity of the mounting device required in the process of attachment and the following capability to the heart shape required in the process of detachment and that furthermore reduces invasion to the patient.

### Solution to Problem

According to one aspect of the present disclosure, there is provided a mounting device used to mount a cardiac net to a heart, wherein the cardiac net has an attachment portion placed in a periphery of an opening of the cardiac net, which serves as an inlet for insertion of the heart into the cardiac net, and provided for attachment of the mounting device. The mounting device comprises a first part and a second part, wherein each of the first part and the second part comprises: a support portion attached to the attachment portion to support the cardiac net in an open state of the opening of the cardiac net; a handle held by a hand; and a connecting structure configured to temporarily connect the first part and the second part with each other.

The mounting device requires, for example, an operation for mounting the cardiac net to the heart after insertion of the cardiac net into the body, an operation for detachment of the mounting device from the cardiac net, and an operation for pulling out the mounting device from the body. The required range of thoracotomy is not only the range required for the insertion but the range required for these operations and accordingly tends to be rather wide.

The mounting device according to the above aspect of the present disclosure, however, has the first part and the second part that are only temporarily connected with each other and that are readily separable from each other. The configuration of this aspect enables the first part and the second part to be separated from each other after the cardiac net is attached to the mounting device, is inserted into the body, and is mounted to the heart. This reduces the constraints in relation to the operation for detachment of the mounting device from the cardiac net and the operation for pulling out the mounting deice from the body. This configuration thus enables these operations to be performed relatively readily even in a narrow range of thoracotomy. Accordingly, the above aspect of the present disclosure enables the cardiac net to be mounted with reducing invasion to the patient.

In the above aspect of the present disclosure, the first part and the second part may be made of any of various materials. It is, however, preferable that a flexible material, such as a resin or a thin metal, is used for the first part and the second part.

The support portion and the handle may be formed integrally or may be provided as separate bodies to be joined with each other.

The cardiac net may be provided with or may not be provided with electrodes. The cardiac net may be provided with a second opening, for example, at a location corresponding to the right ventricle, in addition to the opening that serves as the inlet for insertion of the heart into the cardiac net.

Any of various structures may be employed to the attachment portion for attachment of the mounting device to the cardiac net. For example, a circular configuration like a portion which a rubber cord of underpants is inserted through may be provided in the periphery of the opening by folding back the opening on the upper side of the cardiac net, and the support portion of the mounting device may be inserted in this circular configuration. Instead of this circular configuration, a configuration like a belt loop of pants may be provided outside of the cardiac net, and the support portion of the mounting device may be inserted in this belt loop-like configuration.

In the mounting device of the above aspect, the support portions of the first part and the second part may be respectively curved to form part of a contour of the opening in the open state, and parts of front edges of the respective support portions may be formed in shapes overlapping with each other.

The configuration of this aspect causes the curved portions of the first part and the second part to form the contour of the opening, when the cardiac net is attached to the mounting device. This enables the cardiac net to be opened clearly. This configuration also causes parts of respective front edges of the first part and the second part to overlap with each other. This enables the entire cardiac net to be opened clearly. As a result, this configuration has an advantage that facilitates mounting of the cardiac net to the heart.

In the above aspect, the support portion of each of the first part and the second part may be attached obliquely to the handle, such that the opening is inclined to the handle.

Inclining herein means that an angle between lower faces of the support portion and the handle is neither 0 degree nor 90 degrees. Providing an inclination causes the opening to be in a shape like an inclined butterfly net when the cardiac net is attached to the mounting device. This configuration has an advantage that facilitates the operation of mounting the cardiac net to the heart by an operation of scooping the heart into the cardiac net in the state of thoracotomy from a lateral side of the patient.

In the above aspect, the angle of inclination may be determined arbitrarily and may be, for example, approximately 10 degree.

In the above aspect of the present disclosure, the first part and the second part may have an open-close mechanism configured to be changeable between an open state of a diameter of the opening and a closed state of the diameter of the opening.

The configuration of this aspect enables the cardiac net to be inserted into the body in the closed state of the diameter of the opening of the cardiac net and thereby further reduces invasion to the patient.

This configuration does not intend to exclude a method of inserting the cardiac net into the body in the closed state of the opening by manually warping the first part and the second part that are made of flexible materials. The configuration provided with the above mechanism is, however, more preferable since this configuration enables the opening and closing state of the cardiac net to be freely adjusted even in the body.

The open-close mechanism is not necessarily configured to set the opening in a strictly closed state. There is a requirement that the opening in the closed state is narrowed to be narrower than the opening in the open state.

The open-close mechanism may employ any of various configurations.

In one example, the connecting structures of the first part and the second part may connect the first part and the second part in a rotatable manner with each other at locations of the respective handles and may serve as the open-close mechanism.

For example, a configuration of connecting the first part with the second part on opposite ends thereof that are opposite to the support portions enables the first part and the second part to be opened and closed like tongs. In another example, a configuration of connecting the first part with the second part in the middle of the handles or at a boundary between the handles and the support portions enables the first part and the second part to be opened and closed like scissors.

The connecting structures of the above aspect serving as the open-close mechanism are required to connect the first part and the second part with each other and to separate the first part and the second part from each other after mounting of the cardiac net to the heart. The configuration of providing the connecting structures to open and close the first part and the second part like tongs has an advantage that the connecting structures are not inserted in the body and are thus readily separable from each other.

The configuration of providing the connecting structures to open and close the first part and the second part like scissors, on the other hand, has advantages that suppress an interval between the first part and the second part from being opened widely in the body and thereby reduce the degree of invasion to the body, even in the state that the front edge portions with the net attached thereto are opened.

In this case, the connecting structures may employ any of various configurations. For example, a convex in a columnar shape may be provided in one of the first part and the second part, and a hole which the convex is fit in may be provided in the other of the first part and the second part. This configuration enables these parts to be readily separated from each other by moving the respective parts to be away relative to each other in an axial direction of the convex. In this configuration, formation of the convex and the hole with providing only a limited allowance between the convex and the hole suppresses the first part and the second part from being separated from each other during an operation of mounting the net. In another example, a large diameter portion having a slightly larger diameter may be provided in the vicinity of a top of the convex, with a view to suppressing the separation during the operation. Setting the dimensions of the large diameter portion in a range that allows for elastic deformation of the hole enables the first part and the second part to be separated from each other.

These aspects correspond to an aspect that causes the first part and the second part to be separated from each other by moving the first part and the second part relative to each other in a vertical direction when the first part and the second part are kept horizontally.

The connecting structure may be configured by a mechanism that enables the first part and the second part to be separated from each other by shifting the first part and the second part relative to each other in a longitudinal direction. Furthermore, the connecting structures may be configured by a mechanism that enables the first part and the second part to be separated from each other by opening the first part and the second part to a predetermined angle.

In the mounting device of the above aspect, the second part may be provided with a preventing mechanism configured to prevent the cardiac net attached to the mounting device from shifting from the support portion toward the handle.

The configuration of this aspect enables the cardiac net to be maintained without being shifted from the support portion toward the handle, in the process of pulling out the first part prior to the second part. This configuration accordingly has an advantage that enables the cardiac net to be appropriately maintained at a position where the cardiac net is mounted to the heart.

A variety of structures may be employed for the preventing mechanism. For example, a thicker portion than the support portion or a convex may be provided between the support portion and the handle. It is, however, preferable to configure the preventing mechanism not to interfere with pull-out of the second part from the cardiac net.

The preventing mechanism may have a bending structure formed by bending the support portion of the second part relative to the handle.

The configuration of this aspect suppresses the cardiac net from being shifted by the simple structure. This configuration also has an advantage that such bending does not cause interference when the second part is pulled out from the cardiac net.

Bending herein means that the support portion and the handle are bent at a boundary therebetween or more strictly means that there is a location where derivatives of an outer surface discontinuously change.

In the mounting device according to the above aspect, the first part may have the support portion and the handle that are connected smoothly.

The configuration of this aspect enables the first part to be smoothly pulled out from the cardiac net. This accordingly reduces a possibility that the cardiac net is shifted from a position where the cardiac net is mounted, in the process of pulling out the first part.

The present disclosure is not necessarily provided with all of the variety of features described above but may be implemented by appropriately omitting part of the features or by appropriately combining some of the features.

The present disclosure is not limited to the aspects of the mounting device but may be implemented by a method of mounting a cardiac net using the mounting device.

For example, according to one aspect of the present disclosure, there is provided a method of mounting a cardiac net to a heart. The method of mounting comprises (a) a step of providing a cardiac net that has an attachment portion placed in a periphery of an opening of the cardiac net, which serves as an inlet for insertion of the heart into the cardiac net, and provided for attachment of the mounting device; (b) a step of providing a mounting device that comprises a first part and a second part, wherein each of the first part and the second part comprises: a support portion attached to the attachment portion to support the cardiac net in an open state of the opening of the cardiac net; a handle held by a hand; and a connecting structure configured to temporarily connect the first part and the second part with each other; (c) a step of attaching the cardiac net to the mounting device; (d) a step of inserting the cardiac net with the mounting device into a body of a patient undergoing thoracotomy; (e) a step of, after mounting the cardiac net to the heart, separating the mounting device and pulling out one of the second part and the first part from the body; and (f) pulling out the other of the second part and the first part from the body.

The method of mounting according to this aspect also enables the cardiac net to be mounted with reducing invasion into the patient, as described above with regard to the mounting device of the present disclosure.

A variety of features described above with regard to the mounting device may also be applicable to the method of mounting.

For example, in the method of mounting according to the above aspect, the first part and the second part may have an open-close mechanism configured to be changeable between an open state of a diameter of the opening and a closed state of the diameter of the opening, and the step (d) may comprise inserting the cardiac net with the mounting device in the closed state of the diameter of the opening by the open-close mechanism.

The configuration of this aspect enables the mounting device to be inserted in the closed state into the body and furthermore reduces the invasion.

In the method of mounting according to the above aspect, the second part may have a bending structure formed by bending the support portion relative to the handle, the step (e) may comprise pulling out the first part, and the step (f) may comprise pulling out the second part.

The configuration of this aspect suppresses the cardiac net from being shifted toward the handle of the second part in the process of pulling out the first part after mounting of the cardiac net and enables the position of the mounted cardiac net to be kept appropriately.

### Brief Description of Drawings

Fig. 1 is an explanatory diagram illustrating the state that a cardiac net is attached to a mounting device;
Fig. 2 is an explanatory diagram illustrating the schematic configuration of the cardiac net;
Fig. 3 is an explanatory diagram illustrating the overview of the mounting device;
Fig. 4 is an explanatory diagram illustrating the mounting device separated into a first part and a second part;
Fig. 5 is an explanatory diagram illustrating the first part and the second part in the connected state;
Fig. 6 is an explanatory diagram illustrating the mounting device with the cardia net attached thereto in the closed state;
Fig. 7 is an explanatory diagram illustrating the configuration of the first part;
Fig. 8 is an explanatory diagram illustrating the configuration of the second part;
Fig. 9 is an explanatory diagram illustrating the configuration of a connecting structure of the second part;
Fig. 10 is an explanatory diagram illustrating the configuration of a connecting structure of the first part;
Fig. 11 is explanatory diagrams illustrating a modification (1) of the connecting structures of the first part and the second part;
Fig. 12 is explanatory diagrams illustrating a modification (2) of the connecting structures of the first part and the second part;
Fig. 13 is an explanatory diagram illustrating the state that the cardiac net is being mounted to the heart;
Fig. 14 is an explanatory diagram illustrating the state that the cardiac net is mounted to the heart;
Fig. 15 is an explanatory diagram illustrating the state that the first part of the mounting device is being pulled out;
Fig. 16 is an explanatory diagram illustrating the state that the second part of the mounting device is being pulled out;
Fig. 17 is an explanatory diagram illustrating the state that the cardiac net attached to the mounting device is inserted into a human body model;
Fig. 18 is an explanatory diagram illustrating the state that the cardiac net is mounted to the heart in the human body model;
Fig. 19 is an explanatory diagram illustrating the state that the first part of the mounting device is being pulled out; and
Fig. 20 is an explanatory diagram illustrating the state that the second part of the mounting device is being pulled out.

### Description of Embodiments

The following describes an embodiment of the present disclosure. The description first refers to the configuration of a mounting device according to the embodiment and subsequently refers to a method of mounting a cardiac net to the heart by using the mounting device.

### A. Mounting Device

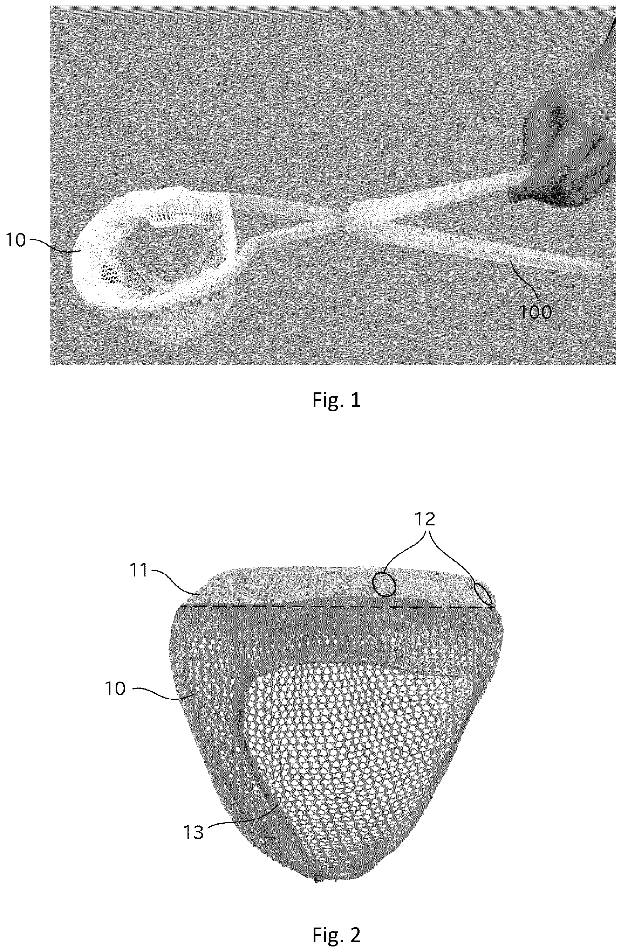

Fig. 1 is an explanatory diagram illustrating the state that a cardiac net is attached to a mounting device. As illustrated, a cardiac net 10 is attached to front edges of a mounting device 100 according to the embodiment.

Fig. 2 is an explanatory diagram illustrating the schematic configuration of the cardiac net. Fig. 2 illustrates the cardiac net in the state that an opening of the cardiac net, which serves as an inlet for insertion of the heart when the cardiac net is mounted to the heart, is placed on an upper side in the drawing. The cardiac net of the embodiment is formed by knitting stretchable fibers. A variety of materials are applicable to the cardiac net 10. It is especially preferable to use biocompatible materials; for example, polyester and polytetrafluoroethylene may be used for the cardiac net 10. Electrodes may be attached to the cardiac net 10 or may be formed in part of the cardiac net 10 by knitting electrically conductive yarns. A variety of methods are employable to knit the cardiac net 10; for example, plain knitting or mesh stitching may be employed.

The cardiac net 10 is also provided with an opening 13 for the right ventricle, such as to cover only the left ventricle when the cardiac net 10 is mounted to the heart. Such an opening 13 may, however, be omitted.

The opening of the cardiac net 10 is partly folded back, so as to form a tubular portion 11 over the entire outer circumference along a broken line in the drawing. The tubular portion 11 has insertion holes 12 that are formed at two locations to insert the mounting device 100 therein. Inserting the front edges of the mounting device 100 through the insertion holes 12 causes the cardiac net 10 to be attached to the mounting device 100 as shown in Fig. 1.

An attachment portion configured to attach the mounting device 100 to the cardiac net 10 may be a configuration like a belt loop of pants, in place of the tubular portion 11.

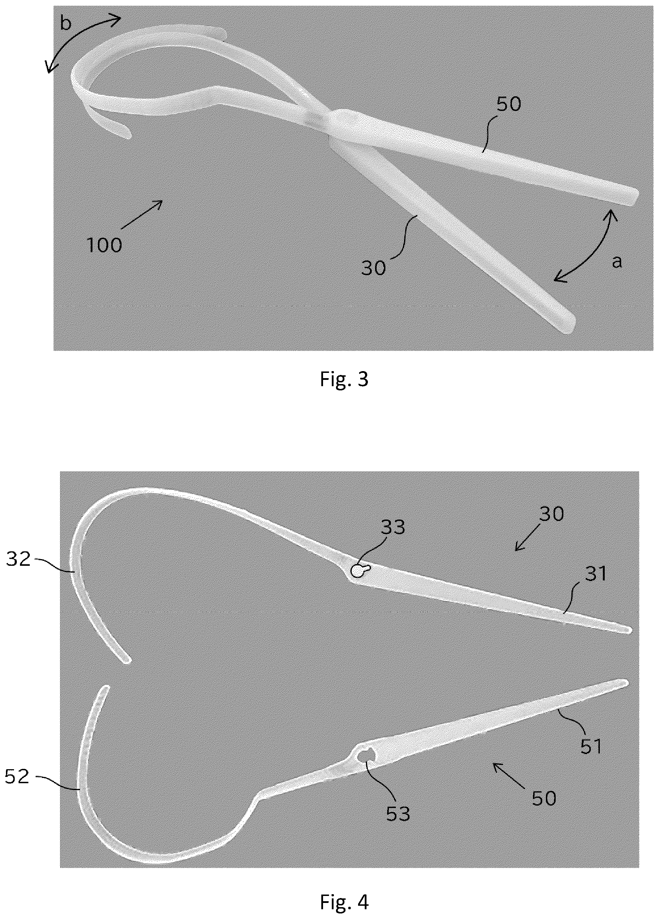

Fig. 3 is an explanatory diagram illustrating the overview of the mounting device. The mounting device 100 of the embodiment is configured by combining a second part 50 with a first part 30. A curved side thereof has portions configured to support the cardiac net. Respective ends of the mounting device on the side which the cardiac net is attached to are configured to be opened and closed like scissors as shown by an arrow b by opening and closing respective ends of handles as shown by an arrow a.

Both the first part 30 and the second part 50 are made of a resin. A variety of materials may be used for the resin. The resin is used for the human body and is thus preferably a material having high biocompatibility and is also preferably a material having flexibility.

Fig. 4 is an explanatory diagram illustrating the mounting device separated into the first part and the second part. Each of the first part 30 and the second part 50 is formed by integral molding of the resin.

The first part 30 includes a handle 31 held by a hand and a support portion 32 curved to support the cardiac net 10. The handle 31 has a convex 33 that is formed to be fit in a hole 53 of the second part.

The second part 50 includes a handle 51 held by the hand and a support portion 52 curved to support the cardiac net 10. The hole 53 is formed in the middle of the handle 51 that configures part of a connection mechanism for connection with the first part 30.

The first part 30 and the second part 50 are connected with each other by laying the first part 30 and the second part 50 one over the other and inserting and fitting the convex 33 into the hole 53. The first part 30 and the second part 50 are readily separated from each other by moving the first part 30 and the second part 50 to be away relative to each other in an axial direction of the convex 33 and pulling out the convex 33 from the hole 53.

The convex 33 and the hole 53 have slightly irregular shapes, because of the reasons described later.

The connection mechanism configured to connect the first part 30 and the second part 50 with each other is not limited to the above configuration but may be any of various mechanisms configured to allow these two parts to be connected with each other and separated from each other. The separation of the first part 30 and the second part 50 from each other is, however, performed in the body of a patient. A preferable mechanism should thus be configured to allow the two parts to be readily separated from each other without applying a heavy load.

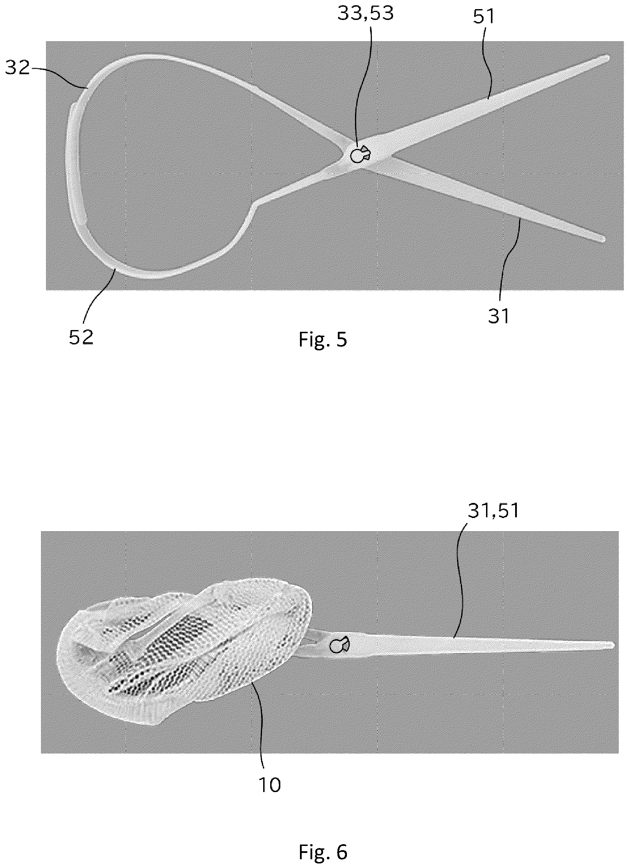

Fig. 5 is an explanatory diagram illustrating the first part and the second part in the connected state. As described above, the first part 30 and the second part 50 are connected with each other by inserting and fitting the convex 33 into the hole 53. The respective support portions 32 and 52 of these two parts are curved and have overlapped ends, such as to form part of the contour of the opening of the cardiac net 10. This configuration enables the mounting device 100 to support the cardiac net 10 with appropriately keeping the opening state.

Fig. 6 is an explanatory diagram illustrating the mounting device with the cardia net attached thereto in the closed state. As described above, when the respective handles 31 and 51 of the first part 30 and the second part 50 are closed, the support portions 32 and 52 on the respective ends are closed like scissors. This configuration allows the cardiac net to be in the closed state as illustrated. This configuration accordingly enables the cardiac net and the mounting device to be inserted into the patient's body with restricting the range of thoracotomy of the patient.

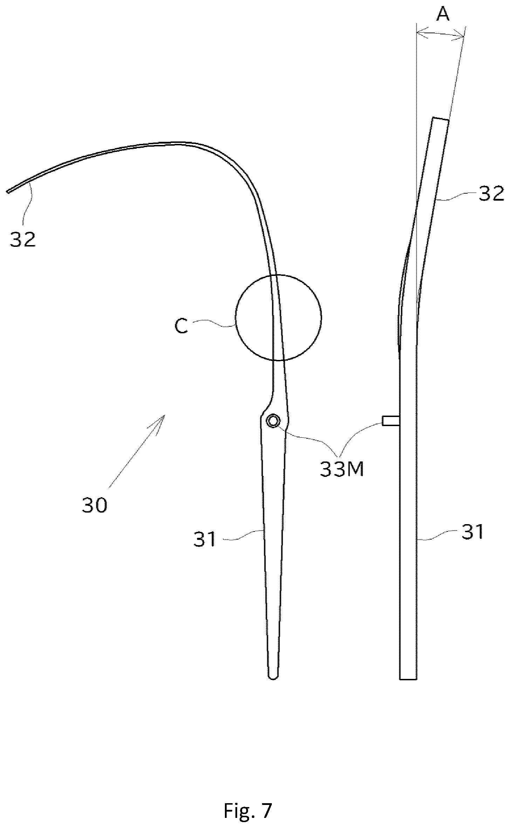

Fig. 7 is an explanatory diagram illustrating the configuration of the first part. The left side drawing is a plan view, and the right side drawing is a side view. As described above, the first part 30 includes the linearly stretched handle 31 and the curved support portion 32. The handle 31 and the support portion 32 are connected smoothly at a boundary C therebetween. This means that the first derivation gives continuous values at any positions on the contour line in the plan view.

As shown in the side view, the support portion 32 is attached to the handle 31 slightly obliquely. An inclination angle A between respective lower faces of the handle 31 and of the support portion 32 is set to 10 degrees. The inclination angle A may be set arbitrarily.

The first part 30 is provided with a convex for connection with the second part 50. Fig. 4 described above illustrates the convex 33 in the irregular shape. Fig. 7 illustrates a convex 33M in a columnar shape as a modification. The convex 33M similarly serves to connect the first part 30 with the second part 50.

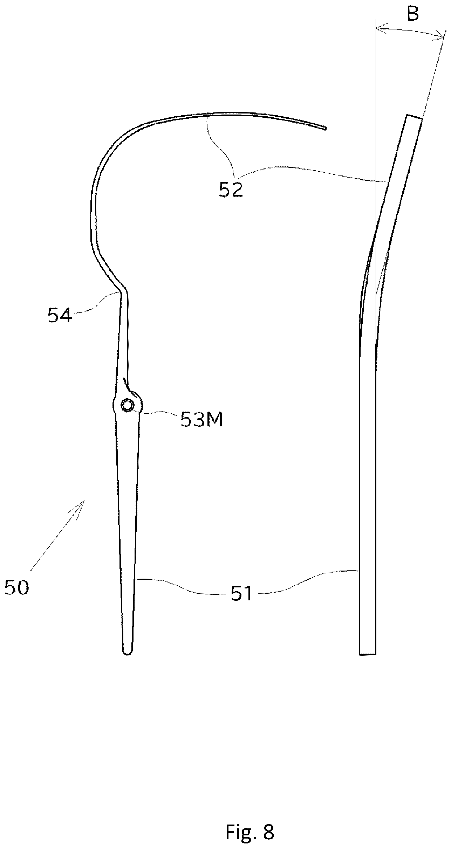

Fig. 8 is an explanatory diagram illustrating the configuration of the second part. The left side drawing is a plan view, and the right side drawing is a side view. As described above, the second part 50 includes the linearly stretched handle 51 and the curved support portion 52. The handle 51 and the support portion 52 have a bent portion 54 at a boundary therebetween. This means that the first derivation of the contour line in the plan view is discontinuous at the bent portion 54. Providing the bent portion 54 has the effect of preventing the cardiac net supported by the support portion 52 from being shifted toward the handle 51.

As shown in the side view, the support portion 52 is attached to the handle 51 slightly obliquely. An inclination angle B between respective lower faces of the handle 51 and of the support portion 52 is set to 10 degrees. The inclination angle B may be set arbitrarily. In order to attach the cardiac net smoothly, however, it is preferable that the inclination angle A of the first part 30 (shown in Fig. 7) is substantially equal to the inclination angle B of the second part 50.

The second part 50 is provided with a hole for connection with the first part 30. Fig. 4 described above illustrates the hole 53 in the irregular shape. Fig. 8 illustrates a hole 53M in a circular shape as a modification. The hole 53M similarly serves to connect the first part with the second part.

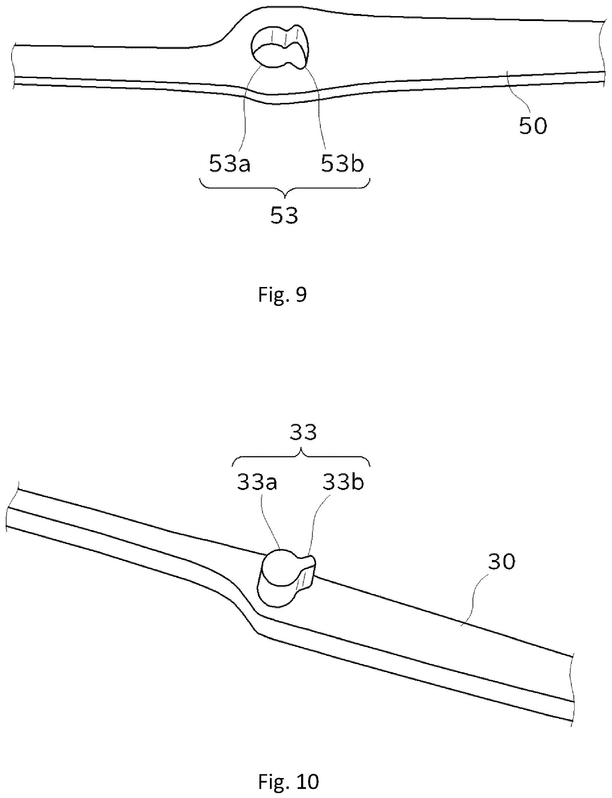

Fig. 9 is an explanatory diagram illustrating the configuration of a connecting structure of the second part. Fig. 9 illustrates the close-up of the hole 53 shown in Fig. 4. The hole 53 has a shape formed by connecting a circular portion 53a with a fan-like portion 53b.

Fig. 10 is an explanatory diagram illustrating the configuration of a connecting structure of the first part. Fig. 10 illustrates the close-up of the convex 33 shown in Fig. 4. The convex 33 has a shape formed by connecting a cylindrical portion 33a with a rectangular columnar portion 33b.

When the convex 33 in this shape is inserted into the hole 53, the cylindrical portion 33a is fit in the circular portion 53a, such as to allow the first part and the second part to rotationally move. The rectangular columnar portion 33b is, on the other hand, movable only inside of the fan-like portion 53b. This configuration restricts the rotations of the first part and the second part. The hole 53 and the convex 33 shown in the embodiment accordingly serve to connect the first part and the second part with each other in a rotatable manner, with restricting the range of the rotations.

The hole 53M (shown in Fig. 8) and the convex 33M shown as the modification may also be used. This modified configuration enables the first part and the second part to be connected with each other in a rotatable manner without limitations. According to the embodiment, the range of rotations of the first part and the second part is naturally restricted by attaching the cardiac net. The mechanism of the modification is thus also be employable.

The following describes modifications of the connecting structures of the first part and the second part.

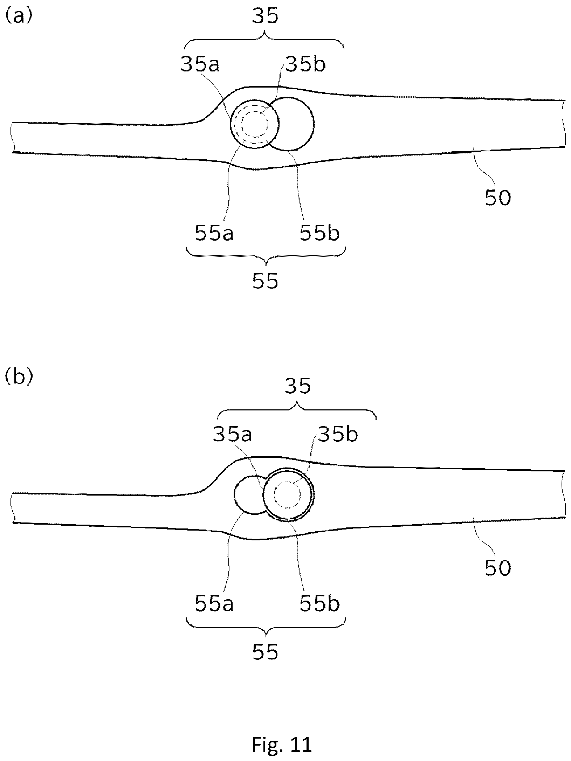

Fig. 11 is explanatory diagrams illustrating a modification (1) of the connecting structures of the first part and the second part. Fig. 11 illustrates planar views in the state that the first part is placed under the second part 50 such as to be fit in the second part 50. For the purpose of avoiding the complication of the illustration, the outer contour of the first part is omitted from the illustration.

According to this modification, a hole 55 formed in the second part 50 has such a shape that a smaller-diameter circular portion 55a and a larger-diameter circular portion 55b are partly overlapped with each other. A convex 35 formed in the first part has a shaft portion 35b in a cylindrical shape and a disk portion 35a formed on a top of the shaft portion 35b. The shaft portion 35b has a diameter in such a range as to be movable between the smaller-diameter circular portion 55a and the larger-diameter circular portion 55b. The disk portion 35a has a diameter in such a range that is larger than the diameter of the smaller-diameter circular portion 55a but that is smaller than the diameter of the larger-diameter circular portion 55b.

As shown in Fig. 11(a), when the convex 35 is located in the smaller-diameter circular portion 55a, the interference of the disk portion 35a causes the first part and the second part to be kept in the connected state.

As shown in Fig. 11(b), when the convex 35 is located in the larger-diameter circular portion 55b by shifting the first part and the second part relative to each other in a longitudinal direction, the disk portion 35a goes through the larger-diameter circular portion 55b. This allows the first part and the second part to be separated from each other.

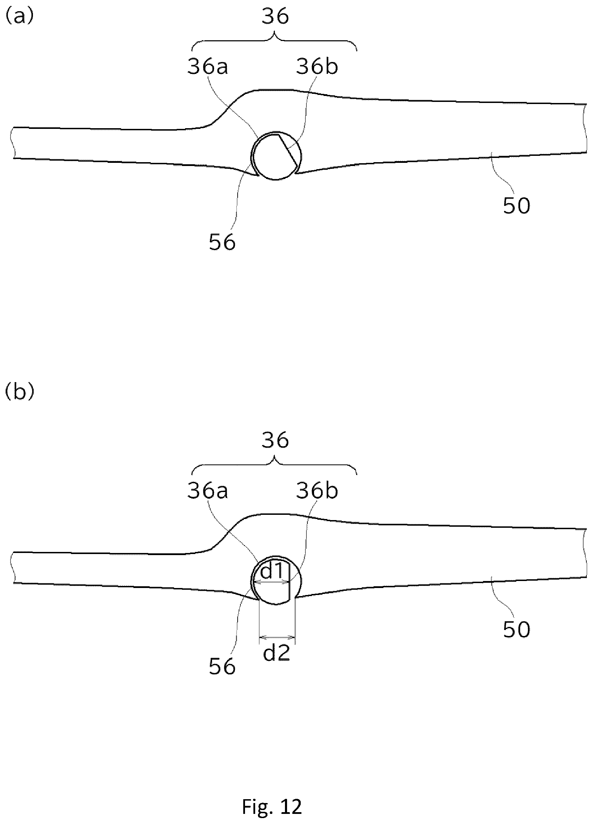

Fig. 12 is explanatory diagrams illustrating a modification (2) of the connecting structures of the first part and the second part. Fig. 12 illustrates planar views in the state that the first part is placed under the second part 50 such as to be fit in the second part 50. For the purpose of avoiding the complication of the illustration, the outer contour of the first part is omitted from the illustration.

According to this modification, a hole 56 is formed, such that an opening is provided in one side of the second part 50. A convex 36 formed in the first part has a cylindrical portion 36a and a planar portion 366b formed by cutting out part of the cylindrical portion 36a. A disk portion having a larger diameter than the diameter of the hole 56 is formed on a top of the convex 36, although this disk portion is omitted from the illustration for the purpose of avoiding the complication of the illustration.

The formation of the planar portion 36b causes a diameter d1 of the convex 36 to become minimum in the state shown in Fig. 12(b). Setting this with d1 to be smaller than a width d2 of the opening of the second part 50 enables the first part and the second part to be separated from each other by opening the first part and the second part to the state shown in Fig. 12(b).

In the case where the first part and the second part are in attitudes other than the state shown in Fig. 12(b), on the other hand, the width of the convex 36 becomes narrower than the width of the opening as shown in Fig. 12(a). The first part and the second part are thus not separated from each other but are kept in the connected state.

Any of other various modified configurations may be employed for the connecting structures.

### B. Method of Mounting Cardiac Net

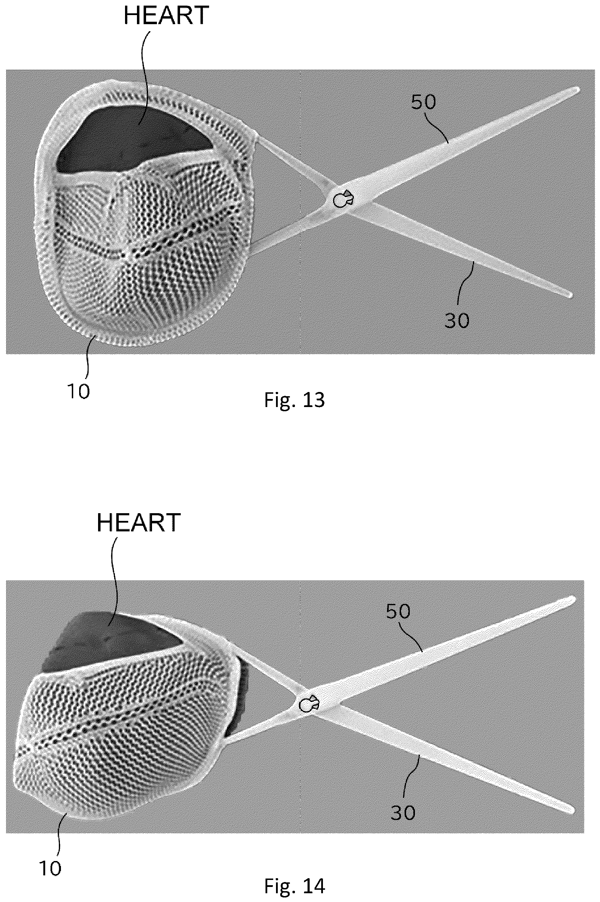

Fig. 13 is an explanatory diagram illustrating the state that the cardiac net is being mounted to the heart. In the illustration, an upper portion of the heart is placed on a back side of the sheet surface, and a lower portion of the heart is placed on a front side. In other words, the illustration shows the heart seen from the lower side. As illustrated, when the first part 30 and the second part 50 are opened, the cardiac net 10 attached to the mounting device is in the opened state and works to scoop the heart upward and to be mounted to the heart.

Fig. 14 is an explanatory diagram illustrating the state that the cardiac net is mounted to the heart. As illustrated, the cardiac net is firmly mounted to the heart up to the top thereof in the state that the first part 30 and the second part 50 are opened. As described above with reference to Fig. 2, the cardiac net 10 of the embodiment has the opening for the right ventricle. In this illustration, part of the heart is exposed on this opening.

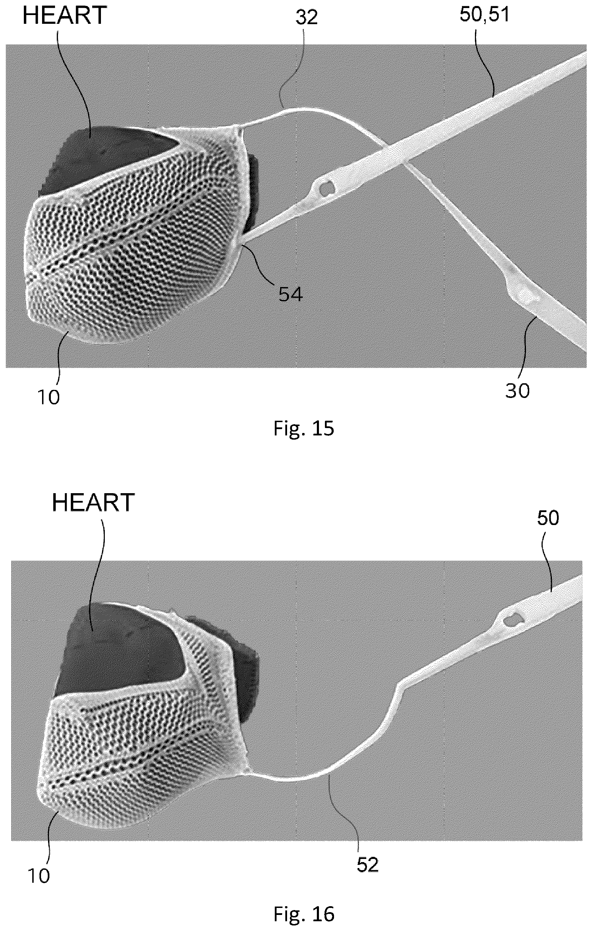

Fig. 15 is an explanatory diagram illustrating the state that the first part of the mounting device is being pulled out. After the cardiac net 10 is mounted to the heart, the second part 50 and the first part 30 are separated from each other, and the first part 30 is pulled out, as illustrated. The support portion 32 of the first part 30 is simply inserted in the cardiac net 10, so that the first part 30 can be readily pulled out by a simple pull. It is preferable that the first part 30 has such a degree of flexibility that facilitates this pull-out.

In the process of pulling out the first part 30, the second part 50 is kept in the state that the cardiac net 10 is attached. The second part 50 is provided with the bent portion 54, so that the cardiac net 10 does not come off toward the handle 51 of the second part 50. The presence of the bent portion 54 has an advantage that the cardiac net 10 is maintained in the appropriate attached state in the process of pulling out the first part 30.

Fig. 16 is an explanatory diagram illustrating the state that the second part of the mounting device is being pulled out. After completion of the pull-out of the first part 30, the second part 50 is pulled out as illustrated. The support portion 52 of the second part 50 is simply inserted in the cardiac net 10, so that the second part 50 can be readily pulled out by a simple pull. It is preferable that the second part 50 has such a degree of flexibility that facilitates this pull-out.

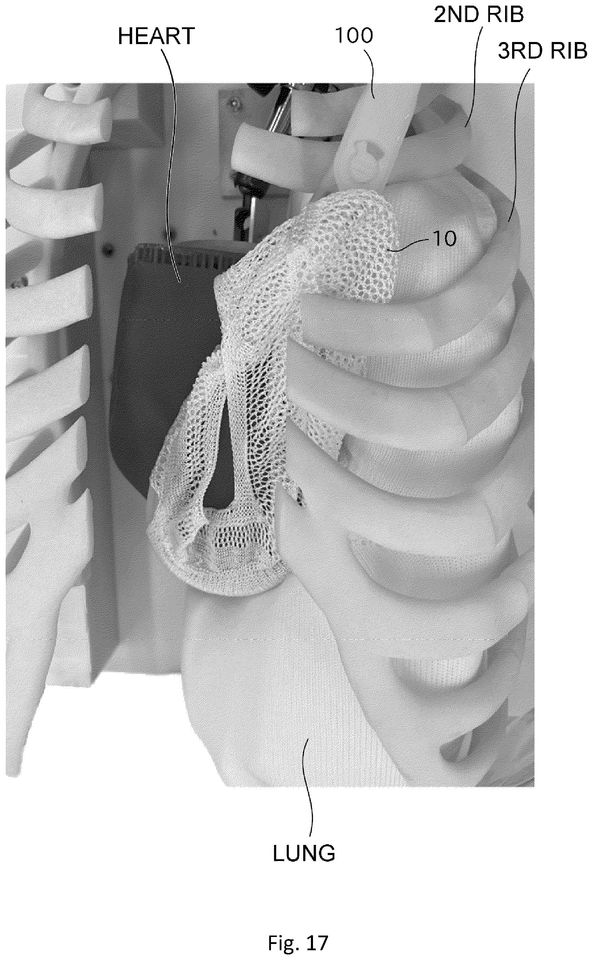

Fig. 17 is an explanatory diagram illustrating the state that the cardiac net attached to the mounting device is inserted into a human body model.

Thoracotomy of a patient is performed at a location corresponding to the second rib and the third rib, and the mounting device 100 is inserted. The mounting device 100 with the cardiac net 10 supported thereby is inserted in the closed state between the second rib and the third rib toward the heart as shown in Fig. 6.

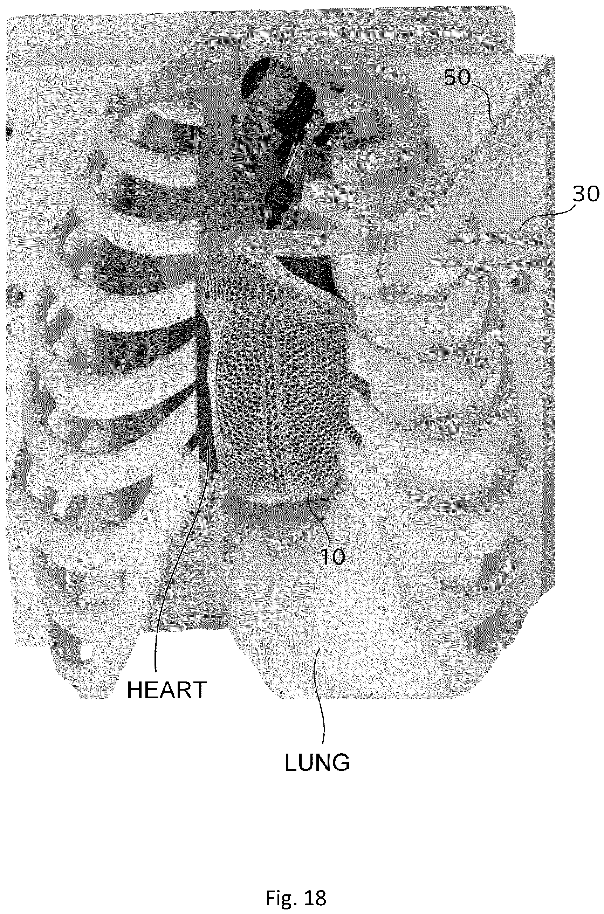

Fig. 18 is an explanatory diagram illustrating the state that the cardiac net is mounted to the heart in the human body model.

After insertion of the mounting device 100 into the body, the first part 30 and the second part 50 are opened, so as to wide open the opening of the cardiac net 10, as illustrated. The cardiac net 10 is then mounted to the heart such as to scoop the heart upward.

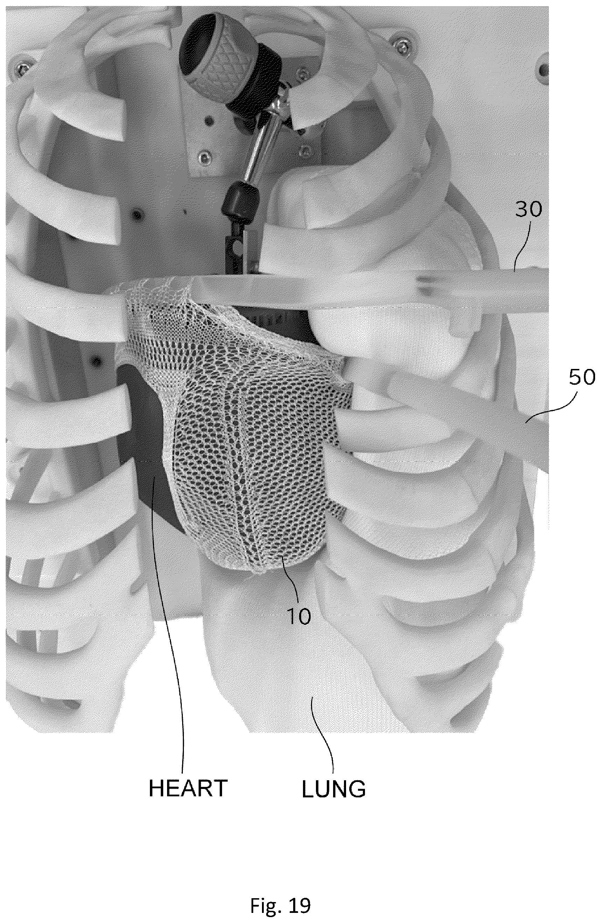

Fig. 19 is an explanatory diagram illustrating the state that the first part of the mounting device is being pulled out.

After completion of mounting of the cardiac net 10, the first part 30 and the second part 50 are separated from each other, and the first part 30 is pulled out.

According to the embodiment, the first part 30 and the second part 50 are separable from each other. This configuration has loose constraint conditions with regard to the attitude of pulling out the mounting device and has an advantage that the mounting device can be sufficiently pulled out even in a narrow range of thoracotomy.

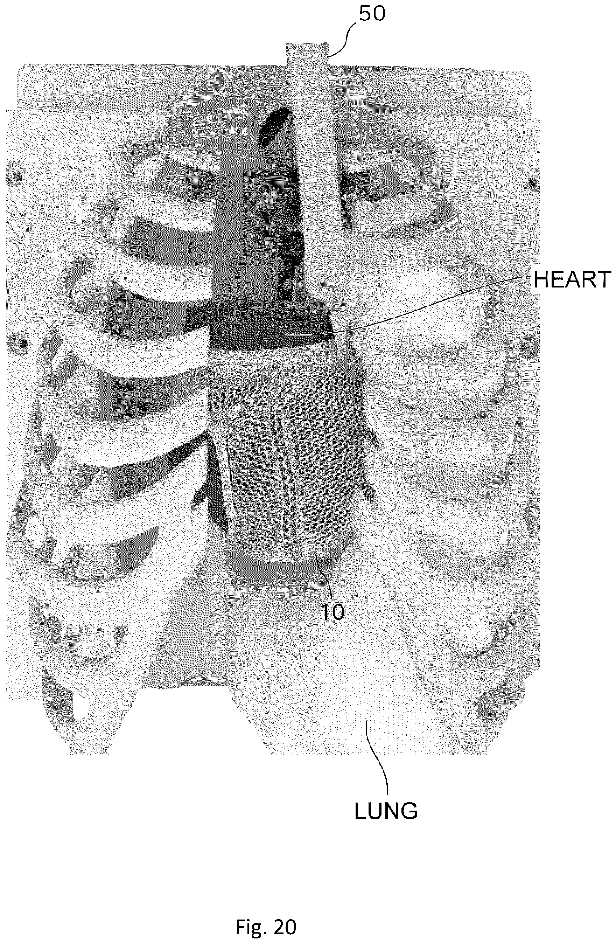

Fig. 20 is an explanatory diagram illustrating the state that the second part of the mounting device is being pulled out.

Eventually pulling out the second part 50 completes mounting of the cardiac net 10.

### C. Advantageous Effects and Modifications

The mounting device and the method of mounting according to the embodiment described above allow the first part and the second part to be separated from each other and accordingly enable the cardiac net to be mounted with reducing the invasion into the patient.

The variety of features provided in the mounting device described in the embodiment enable the method of mounting the cardiac net to be performed more readily and appropriately.

The present disclosure is not necessarily provided with all of the variety of features described in the embodiment but may be implemented by appropriately omitting part of the features or by appropriately combining some of the features.

The present disclosure is not limited to the embodiment but may be configured by a variety of modifications.

### Industrial Applicability

The present disclosure is applicable to a mounting device of a cardiac net and a method of mounting the cardiac net.

### Reference Signs List

10 cardiac net
11 tubular portion
12 insertion hole
13 opening
30 first part
31, 51 handles
32, 52 support portions
33, 33M convexes
33a, 33b portions
35 convex
35a disk portion
35b shaft portion
36 convex
36a, 36b portions
50 second part
52 support portion
53, 53M holes
53a, 53b portions
54 bent portion
55 hole
55a, 55b portions
56 hole
100 mounting device

## Claims

1. A mounting device used to mount a cardiac net to a heart, wherein
the cardiac net has an attachment portion placed in a periphery of an opening of the cardiac net, which serves as an inlet for insertion of the heart into the cardiac net, and provided for attachment of the mounting device,
the mounting device comprising a first part and a second part, wherein
each of the first part and the second part comprises:
a support portion attached to the attachment portion to support the cardiac net in an open state of the opening of the cardiac net;
a handle held by a hand; and
a connecting structure configured to temporarily connect the first part and the second part with each other.

2. The mounting device according to claim 1,
wherein the support portions of the first part and the second part are respectively curved to form part of a contour of the opening in the open state, and
parts of front edges of the respective support portions are formed in shapes overlapping with each other.

3. The mounting device according to claim 2,
wherein the support portion of each of the first part and the second part is attached obliquely to the handle, such that the opening is inclined to the handle.

4. The mounting device according to claim 1,
wherein the first part and the second part have an open-close mechanism configured to be changeable between an open state of a diameter of the opening and a closed state of the diameter of the opening.

5. The mounting device according to claim 4,
wherein the connecting structures of the first part and the second part connect the first part and the second part in a rotatable manner with each other at locations of the respective handles and serve as the open-close mechanism.

6. The mounting device according to claim 1,
wherein the second part is provided with a preventing mechanism configured to prevent the cardiac net attached to the mounting device from shifting from the support portion toward the handle.

7. The mounting device according to claim 6,
wherein the preventing mechanism has a bending structure formed by bending the support portion of the second part relative to the handle.

8. The mounting device according to claim 1,
wherein the first part has the support portion and the handle that are connected smoothly.

9. A method of mounting a cardiac net to a heart, comprising:
(a) a step of providing a cardiac net that has an attachment portion placed in a periphery of an opening of the cardiac net, which serves as an inlet for insertion of the heart into the cardiac net, and provided for attachment of the mounting device;
(b) a step of providing a mounting device that comprises a first part and a second part, wherein each of the first part and the second part comprises: a support portion attached to the attachment portion to support the cardiac net in an open state of the opening of the cardiac net; a handle held by a hand; and a connecting structure configured to temporarily connect the first part and the second part with each other;
(c) a step of attaching the cardiac net to the mounting device;
(d) a step of inserting the cardiac net with the mounting device into a body of a patient undergoing thoracotomy;
(e) a step of, after mounting the cardiac net to the heart, separating the mounting device and pulling out one of the second part and the first part from the body; and
(f) pulling out the other of the second part and the first part from the body.

10. The method of mounting according to claim 9,
wherein the first part and the second part have an open-close mechanism configured to be changeable between an open state of a diameter of the opening and a closed state of the diameter of the opening, and
the step (d) comprises inserting the cardiac net with the mounting device in the closed state of the diameter of the opening by the open-close mechanism.

11. The method of mounting according to claim 9,
wherein the second part has a bending structure formed by bending the support portion relative to the handle,
the step (e) comprises pulling out the first part, and
the step (f) comprises pulling out the second part.
